# EUROPEAN PATENT SPECIFICATION

(11) **EP 3 545 826 B1**
(45) Date of publication and mention of the grant of the patent: **17.09.2025**
(21) Application number: 19166164.4
(22) Date of filing: 29.03.2019
(51) Int. Cl.: A61B 5/00, A61B 5/1455

(54) **SUPPORT TOOL**
UNTERSTÜTZUNGSWERKZEUG
OUTIL DE SUPPORT

(30) Priority: 30.03.2018 JP 2018067618
(43) Date of publication of application: 02.10.2019
(73) Proprietor: Nihon Kohden Corporation, Tokyo 161-8560 (JP)
(72) Inventor: SAEKI, Kota, Tokorozawa-shi, Saitama (JP); JIANG, Shen, Cambridge, MA 02139 (US); WEISNER, Steve, Cambridge, MA 02139 (US)
(74) Representative: Grünecker Patent- und Rechtsanwälte PartG mbB

(56) References cited:
- EP-A1- 0 481 612
- EP-A1- 3 117 769
- EP-B1- 0 481 612
- WO-A2-98/04182
- US-A- 5 339 810
- US-A- 5 368 025
- US-A1- 2002 165 440
- US-A1- 2007 027 376
- US-A1- 2013 048 194
- US-B2- 6 916 289

## Description

The presently disclosed subject matter relates to a tool which is to be attached to the finger of a subject to support acquisition of physiological parameter of the subject that is performed by a sensor.

An example of such a sensor is a probe for a pulse oximeter. A probe having an emitter and a detector is attached to the fingertip of the subject, and used for acquiring physiological parameter such as the non-invasive arterial oxygen saturation (SpO2).

WO/2015/137151 discloses an example of a support tool which is used together with such a sensor. The support tool includes first and second support members. The first support member is placed on a first side of the finger of the subject. The second support member is placed on a second side of the finger which is opposite to the first side. The first and second support members are formed by a rigid resin, and their relative positions are made unchangeable. The support tool further includes a bag member which is supported by the first support member. The bag member is formed by a material which is more flexible than the first and second support members. The support tool further includes a fluid passage which communicates with the interior of the bag member. Other support tool designs including bag members are disclosed in EP3117769, WO98/04182 and US6916289.

When the air is sent into the bag member through the fluid passage, the bag member inflates. The inflation of the bag member causes the finger of the subject to be pressed, and the blood is excluded from the tissue.

When air evacuation is performed through the fluid passage, the bag member deflates. This releases the pressing, and the blood returns to the tissue. The capillary refilling time can be specified as physiological parameter of the subject from a change in reception light intensity before and after the pressing.

It is an object of the presently disclosed subject matter to, in acquisition of physiological parameter which involves pressing of the finger of the subject as described above, reduce the burden on the finger while miniaturizing a tool for supporting the acquisition.

### SUMMARY OF INVENTION

According to an aspect of the invention, a support tool which is to be attached to a finger of a subject, and which is used for supporting acquisition of physiological parameter of the subject, includes a support member that is to be placed on a first side of the finger; a bag member that is to be placed on a second side of the finger that is opposite to the first side; a fluid passage that communicates with an interior of the bag member; and a band portion that is constraining the bag member to the finger, wherein a sensor used for acquiring the physiological parameter is placeable at least one of between the finger and the support member, and between the finger and the bag member, wherein the support member includes a hinge portion which is continuous to the bag member, wherein the hinge portion is bendable, and wherein the bag member includes: a first portion that is molded integrally with the support member; and a second portion that is welded to the first portion such that the first portion and the second portion define an interior of the bag member therebetween.

According to the configuration, as compared with, for example, the configuration disclosed in Patent Literature 1, one of the pair of support members which are placed respectively on the both sides of the finger can be omitted. Therefore, the size and weight of the support tool can be reduced. Accordingly, also the burden on the finger of the subject to which the support tool is attached can be reduced. On the other hand, change of the relative position with respect to the support member can be prevented while allowing the bag member to deform, and therefore decrease of the accuracy of acquiring the physiological parameter due to the miniaturization and the weight reduction can be prevented.

### BRIEF DESCRIPTION OF DRAWINGS

FIG. 1 illustrates the appearance of a support tool of an embodiment.
FIG. 2 illustrates a sectional structure of the support tool.
FIGS. 3A and 3B illustrate a manner of attaching the support tool to a finger.
FIGS. 4A and 4B illustrate manners of attaching a support tool to a finger.
FIG. 5 illustrates a configuration example of the bag member of the support tool.
FIG. 6 illustrates a modification of the support tool.

### DETAILED DESCRIPTION OF EMBODIMENTS

Hereinafter, an embodiment will be described in detail with reference to the accompanying drawings. In the drawings, in order to make the components to be described, to have a recognizable size, their scales are appropriately changed.

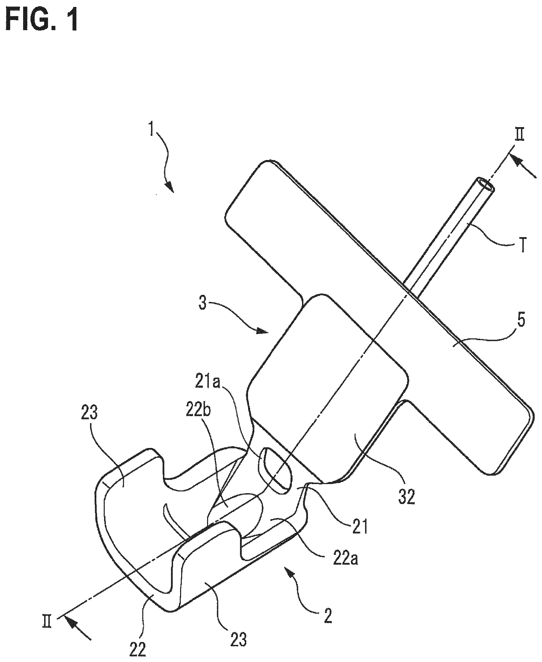

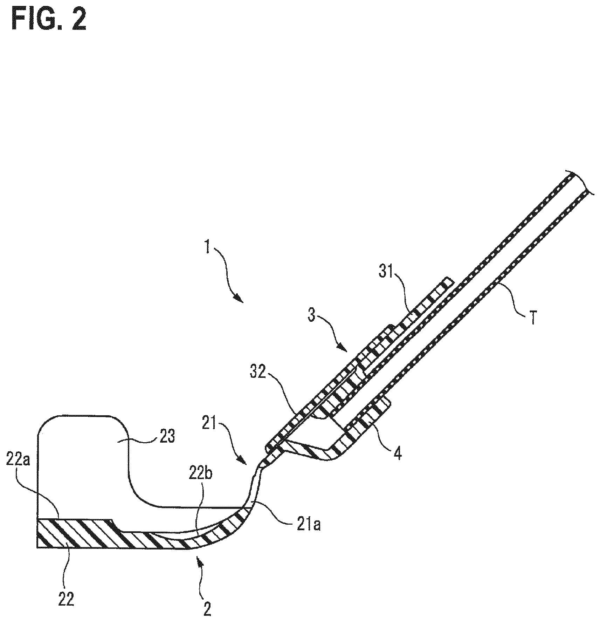

FIG. 1 illustrates the appearance of a support tool 1 of an embodiment. FIG. 2 illustrates a section of the support tool 1 taken along line II-II as viewed in the direction of the arrows.

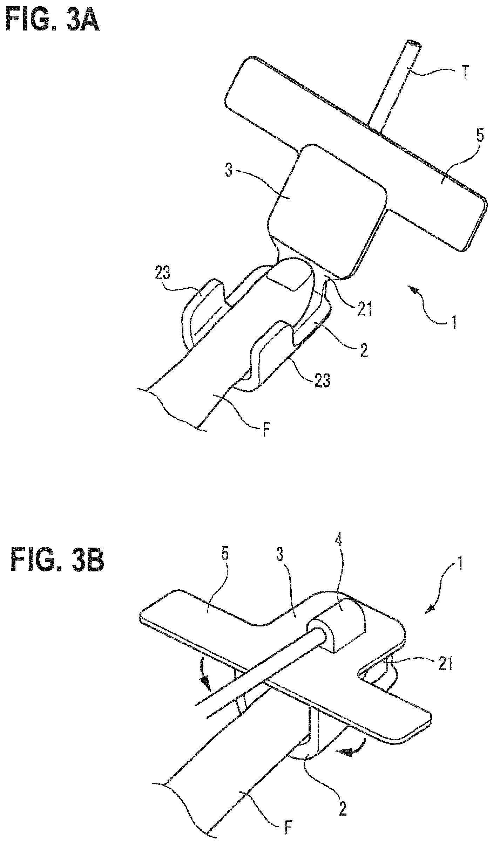

The support tool 1 is a tool which is to be attached to the finger of the subject, and which is used for supporting acquisition of physiological parameter of the subject that is performed by a sensor. The support tool 1 may include a support member 2. The support member 2 is made of a resin. As illustrated in of FIG. 3A, when the support tool 1 is attached to the finger F of the subject, the support member 2 is placed on the pad side of the finger F. The pad side of the finger F is an example of the first side of the finger.

The support tool 1 may include a bag member 3 and a fluid passage 4. As illustrated in FIG. 2, the fluid passage 4 communicates with the interior of the bag member 3. A tube T can be connected to the fluid passage 4. The tube T is connected to a pump apparatus which is not illustrated. As illustrated in of FIG. 3B, when the support tool 1 is attached to the finger F of the subject, the bag member 3 is placed on the nail side of the finger F. The nail side of the finger F is an example of the second side of the finger.

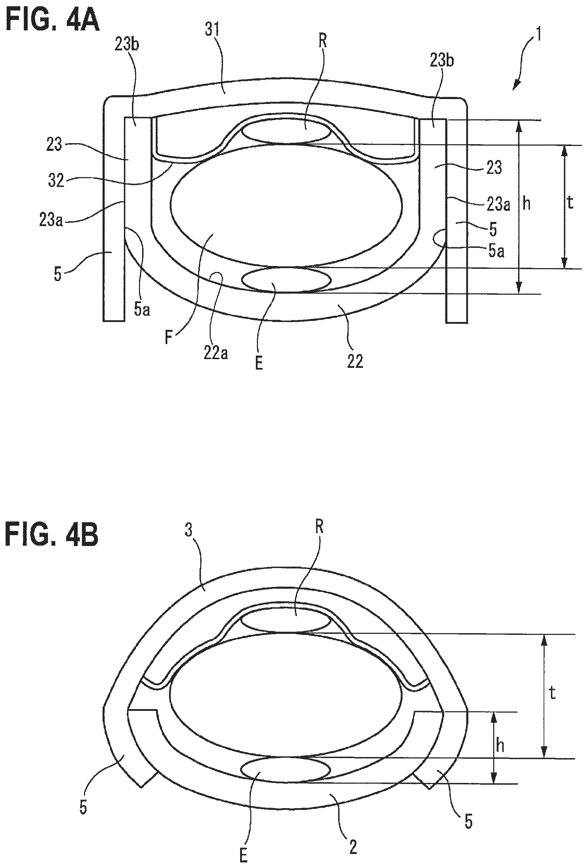

FIG. 4A schematically illustrates a state of the support tool 1 attached of the finger F as viewed from the base of the finger F. The sensor in the embodiment is a probe for a so-called pulse photometer, and may include a light emitter E and a light detector R. The light emitter E is placed between the finger F and the support member 2. The light detector R is placed between the finger F and the bag member 3.

The bag member 3 is more flexible than the support member 2. When a fluid such as the air is sent into the bag member 3 through the fluid passage 4, the bag member 3 inflates. The inflation of the bag member 3 causes the finger F to be pressed, and the blood is excluded from the tissue. When air evacuation is performed through the fluid passage 4, the bag member 3 deflates. This releases the pressing, and the blood returns to the tissue. The capillary refilling time can be specified as physiological parameter of the subject from a change in reception light intensity in the light detector R before and after the pressing. A detailed description of the technique for specifying the capillary refilling time by using a probe of a pulse photometer is omitted.

The support tool 1 may include a band portion 5. When the support tool 1 is attached to the finger F of the subject, the band portion 5 constrains the bag member 3 to the finger F.

According to the configuration, as compared with, for example, the configuration disclosed in WO/2015/137151, one of the pair of support members which are placed respectively on the both sides of the finger can be omitted, and therefore the size and weight of the support tool 1 can be reduced. Accordingly, also the burden on the finger F of the subject to which the support tool 1 is attached can be reduced. On the other hand, change of the relative position with respect to the support member 2 can be prevented from, while allowing the bag member 3 to deform, and therefore decrease of the accuracy of acquiring the physiological parameter due to the miniaturization and the weight reduction can be prevented.

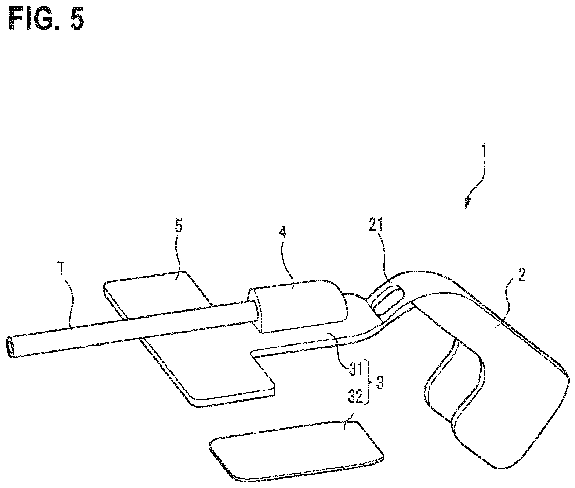

As illustrated in FIG. 5, the bag member 3 may include a first portion 31 and a second portion 32. In this case, the first portion 31 is molded integrally with the support member 2. Namely, the first portion 31 is formed by the same resin material as the support member 2. As illustrated in FIG. 2, the second portion 32 is welded to the first portion 31. Therefore, the interior space of the bag member 3 which communicates with the fluid passage 4 is defined. The thickness of the first portion 31 and the material forming the second portion 32 are appropriately selected so that a desired deformability is attained in the bag member 3.

Specifically, the support member 2 may include a thin hinge portion 21. The first portion 31 of the bag member 3 is continuous to the hinge portion 21. As illustrated in FIGS. 3A and 3B, the bag member 3 is allowed to be placed on the nail side of the finger F, simply by bending the hinge portion 21 in a state where the pad side of the finger F of the subject is placed on the support member 2.

According to the configuration, a state where the physiological parameter can be acquired by the sensor can be easily obtained while ensuring the desired deformability of the bag member 3. Namely, the accuracy of the acquisition of the physiological parameter can be prevented from being lowered, while enhancing the convenience of the user.

As illustrated in FIG. 2A and FIG. 4, the second portion 32 of the bag member 3 is thinner than the first portion 31.

According to the configuration, the bag member 3 can be provided with high flexibility irrespective of the material forming the second portion 32. When the bag member 3 inflates, therefore, the contact to the finger F of the subject can be enhanced, and the accuracy of the acquisition of the physiological parameter can be prevented from being lowered.

The support member 2 may include a bottom wall 22 and a pair of side walls 23. As illustrated in FIG. 3A and FIG. 4A, in this case, the finger F of the subject is placed between the pair of side walls 23. At this time, the inner surface 22a of the bottom wall 22 is opposed to the pad side of the finger F. One of hook and loop portions (not shown) of a surface fastener may be placed on each of the outer side surfaces 23a of the pair of side walls 23.

The band portion 5 is molded integrally with the first portion 31 of the bag member 3. The other of the hook and loop portions (not shown) of a surface fastener may be placed on the inner surface 5a of the band portion 5. When the surface fastener on the band portion 5 is contacted with the surface fastener portions of the side walls 23, the band portion 5 is fixed to the support member 2.

Alternatively, each of the outer side surfaces 23a of the pair of side walls 23 may be formed as an adhering surface in which an adhesive agent layer is formed. In the alternative, when the inner surface 5a of the band portion 5 is contacted with the outer side surfaces 23a, the band portion 5 is fixed to the support member 2.

Since the band portion 5 is molded integrally with the bag member 3, the number of components can be reduced, and furthermore a state where physiological parameter can be acquired by the sensor is easily obtained simply by winding the band portion 5 around the support member 2 in the state illustrated in FIG. 3B. Therefore, the convenience of the user is improved.

A configuration may be employed where the structure corresponding to the pair of side walls 23 is disposed on the side of the bag member 3, and the band portion 5 is molded integrally with the support member 2.

As illustrated in FIG. 4A, the height h of the pair of side walls 23 is larger than the thickness t of a part of the finger F which is placed between the pair of side walls 23.

According to the configuration, the burden on the finger F of the subject can be largely reduced, and the accuracy of the acquisition of the physiological parameter can be prevented from being lowered. The reason of this will be described with reference to a comparison example illustrated in FIG. 4B.

As shown in FIG. 4B, in the state where the bag member 3 is constrained to the finger F by the band portion 5, a state where the band portion 5 is stretched between the pair of side walls 23 is formed. Namely, a tension is generated in the band portion 5. In the case where the height h of the pair of side walls 23 is smaller than the thickness t of the finger F as in the comparison example, the tension of the band portion 5 acts also on the finger F, and therefore the finger F continues to be pressed irrespective of the state of the bag member 3. Consequently, a burden is imposed on the finger F, and furthermore the pressing force which is exerted when the bag member 3 inflates changes in accordance with the manner of fixing the band portion 5, thereby impeding correct acquisition of physiological parameter.

According to the configuration of the embodiment, as illustrated in FIG. 4A, on the other hand, the tension of the band portion 5 is received by upper end portions 23b of the pair of side walls 23, and therefore the tension can be prevented from being exerted on the finger F. During the period when the finger F is pressed due to the inflation of the bag member 3, an influence of the tension can be eliminated. Therefore, the burden on the finger F during attachment of the support tool 1 can be reduced, and furthermore the accuracy of the acquisition of the physiological parameter can be prevented from being lowered.

A hook portion (not shown) of a surface fastener may be placed on the inner surface 22a of the bottom wall 22 of the support member 2 which is opposed to the pad side of the finger F of the subject.

In the case where a part of the light emitter E is covered by fibers, the fibers function as a loop portion of a surface fastener, and a relative displacement between the support member 2 and the light emitter E during attachment of the support tool 1 is suppressed. Therefore, the accuracy of the acquisition of the physiological parameter can be prevented from being lowered. In the case where a hook portion of a surface fastener is placed on a part of the surface of the light emitter E, the surface fastener which is placed on the inner surface 22a of the bottom wall 22 can be set to have a loop portion.

Alternatively, the inner surface 22a of the bottom wall 22 may be formed as an adhering surface in which an adhesive agent layer is formed. Also in such a configuration, a relative displacement between the support member 2 and the light emitter E during attachment of the support tool 1 can be suppressed.

Same or similarly, a hook portion (not shown) of a surface fastener may be placed on the surface of the second portion 32 of the bag member 3 which is opposed to the nail side of the finger F of the subject.

In the case where a part of the light detector R is covered by fibers, the fibers function as a loop portion of a surface fastener, and a relative displacement between the bag member 3 and the light detector R during attachment of the support tool 1 is suppressed. Therefore, the accuracy of the acquisition of the physiological parameter can be prevented from being lowered. In the case where a hook portion of a surface fastener is placed on a part of the surface of the light detector R, a surface fastener which is placed on the surface of the second portion 32 of the bag member 3 can be set to have a loop portion.

Alternatively, the surface of the second portion 32 of the bag member 3 may be formed as an adhering surface in which an adhesive agent layer is formed. Also in such a configuration, a relative displacement between the bag member 3 and the light detector R during attachment of the support tool 1 can be suppressed.

As illustrated in FIGS. 1 and 2, a concave portion 22b may be formed in the inner surface 22a of the bottom wall 22 of the support member 2 which is opposed to the pad portion of the finger F of the subject.

According to the configuration, at least a part of the light emitter E which is placed between the finger F of the subject and the support member 2 is received by the concave portion 22b. Therefore, the feeling of pressure on the finger F due to the light emitter E can be suppressed, and also the relative displacement between the support member 2 and the light emitter E during attachment of the support tool 1 can be suppressed. Therefore, not only the burden on the finger F during attachment of the support tool 1 can be suppressed, but also the lowering of the accuracy of the acquisition of the physiological parameter can be suppressed.

As illustrated in FIGS. 1 and 2, an opening 21a is formed in the hinge portion 21 of the support member 2. In the state where the support tool 1 is attached to the finger F of the subject as illustrated in FIG. 3B, a portion of the fingertip can be seen through the opening 21a. The fingertip portion is an example of the part of the finger.

According to the configuration, even when, during attachment of the support tool 1, the finger F of the subject is covered by the support member 2, the bag member 3, and the band portion 5, the color of the finger F can be checked through the opening 21a. Therefore, a prompt countermeasure may be enabled in the case where the band portion 5 is accidentally tightly wound to cause congestion. Consequently, the burden on the finger F during attachment of the support tool 1 can be suppressed.

The above-described embodiment is a mere example for facilitating understanding of the presently disclosed subject matter.

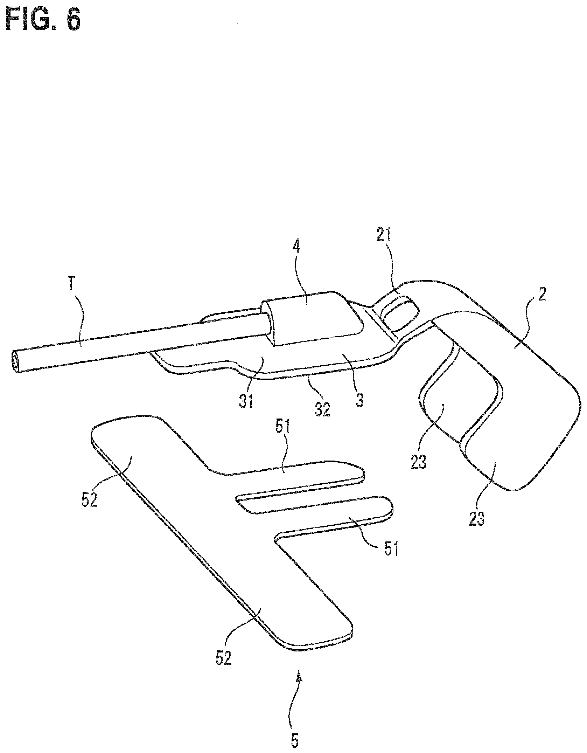

In the embodiment, the band portion 5 is molded integrally with the first portion of the bag member 3. As illustrated in FIG. 6, however, the band portion 5 may be configured as an independent member. In this case, the band portion 5 may include a first portion 51 and a second portion 52. The first portion 51 is fixed to the second portion 32 of the bag member 3. The fixation may be performed by forming an adhesive agent layer on the first portion 51, or by disposing surface fasteners on the first portion 51 and the second portion 52, respectively. The second portion 52 is fixed to the pair of side walls 23 of the support member 2 by the above-described technique.

According to the configuration, when only the band portion 5 is replaced with a new one, the support member 2 and the bag member 3 can be reused.

Also in the example illustrated in FIG. 6, a configuration where the structure corresponding to the pair of side walls 23 is disposed on the side of the bag member 3 may be employed. In this case, the first portion 51 of the band portion 5 is fixed to the support member 2, and the second portion 52 of the band portion 5 is fixed to the bag member 3.

In the above-described embodiment, the band portion 5 is configured by a pair of band-like members which extend from the side parts of the first portion 31 of the bag member 3, respectively. Alternatively, the band portion 5 may be configured by a single band-like member which extends from one side part of the first portion 31. In the alternative, the single band-like member is wound so as to extend from one side wall 23 to the other side wall 23 through the bottom wall 22.

In the above-described embodiment, the light emitter E and the light detector R are placed on the pad and nail sides of the finger F, respectively. However, the light emitter E and the light detector R may be placed on the nail and pad sides of the finger F, respectively. Alternatively, both the light emitter E and the light detector R may be placed on one of the nail and pad sides of the finger F.

## Claims

1. A support tool (1) which is to be attached to a finger (F) of a subject, and which is used for supporting acquisition of physiological parameter of the subject, comprising:
a support member (2) that is to be placed on a first side of the finger (F);
a bag member (3) that is to be placed on a second side of the finger (F) that is opposite to the first side;
a fluid passage (4) that communicates with an interior of the bag member (3); and
a band portion (5) constraining the bag member (3) to the finger (F),
wherein a sensor used for acquiring the physiological parameter is placeable at least one of between the finger (F) and the support member (2), and between the finger (F) and the bag member (3),
**characterized in that** the support member (2) includes a hinge portion (21) which is continuous to a first portion (31) of the bag member (3),
wherein the hinge portion (21) is bendable, and
wherein the bag member (3) includes:
the first portion (31), wherein the first portion (31) is molded integrally with the support member (2); and
a second portion (32) that is welded to the first portion (31) such that the first portion and the second portion define an interior of the bag member therebetween.

2. The support tool according to claim 1, wherein the second portion (32) is thinner than the first portion (31).

3. The support tool according to any one of claims 1 or 2, wherein the band portion (5) is molded integrally with one of the support member (2) and the bag member (3), and fixable to another one of the support member (2) and the bag member (3).

4. The support tool according to any one of claims 1 or 2, wherein the band portion (5) is separate member from the support member (2) and the bag member (3), and includes:
a first portion (51) that is fixable to one of the support member (2) and the bag member (3); and
a second portion (52) which is fixable to another one of the support (2) member and the bag member (3).

5. The support tool according to any one of claims 1 to 4, wherein the support member (2) has a pair of side walls (23) between which a part of the finger (F) is to be placed, and
a height (h) of the pair of side walls (23) is a height at which a light emitter (E) and a light detector (R) are positioned in a cross-sectional shape of the support member (2).

6. The support tool according to any one of claims 1 to 5, wherein an adhesive surface or a surface fastener is placed on a surface of at least one of the support member (2) and the bag member (3), the surface being opposed to the finger (F).

7. The support tool according to any one of claims 1 to 6, wherein a concave portion (22b) is formed on a surface (22a) of the support member (2), the surface (22a) being opposed to the finger (F).

8. The support tool according to any one of claims 1 to 7, wherein the support member (2) has an opening (21a) through which a part of the finger (F) is seen when the support member (F) is attached to the finger (F).

9. The support tool according to claim 1, wherein the bag member (3) includes:
the first portion (31) being formed by same resin material as the resin material of the support member (2).

10. A sensor with supporting tool comprising:
a sensor that is configured to acquire physiological parameter of the subject; and
a support tool according to claim 1.

## Patentansprüche

1. Unterstützungsvorrichtung (1), die an einem Finger (F) eines Patienten anzubringen ist und die dazu dient, Erfassung eines physiologischen Parameters des Patienten zu unterstützen, wobei sie umfasst:
ein Trageelement (2), das an einer ersten Seite des Fingers (F) zu positionieren ist;
ein Schlauchelement (3), das an einer zweiten Seite des Fingers (F) zu positionieren ist, die der ersten Seite gegenüberliegt;
einen Fluiddurchlass (4), der mit einem Innenraum des Schlauchelementes (3) in Verbindung steht; sowie
einen Bandabschnitt (5), der das Schlauchelement (3) an dem Finger (F) festhält, wobei ein Sensor, der zum Erfassen des physiologischen Parameters dient, zwischen dem Finger (F) und dem Trageelement (2) oder/und zwischen dem Finger (F) und dem Schlauchelement (3) positioniert werden kann,
**dadurch gekennzeichnet, dass**
das Trageelement (2) einen Gelenkabschnitt (21) enthält, der sich an einen ersten Abschnitt (31) des Schlauchelementes (3) anschließt,
wobei der Gelenkabschnitt (21) gebogen werden kann, und
wobei das Schlauchelement (3) enthält:
den ersten Abschnitt (31), wobei der erste Abschnitt (31) integral mit dem Trageelement (2) geformt ist; sowie
einen zweiten Abschnitt (32), der an dem ersten Abschnitt (31) so angeschweißt ist, dass zwischen dem ersten Abschnitt und dem zweiten Abschnitt ein Innenraum des Schlauchelementes gebildet wird.

2. Unterstützungsvorrichtung nach Anspruch 1, wobei der zweite Abschnitt (32) dünner ist als der erste Abschnitt (31).

3. Unterstützungsvorrichtung nach einem der Ansprüche 1 oder 2, wobei der Bandabschnitt (5) integral mit dem Trageelement (2) oder dem Schlauchelement (3) geformt ist und an dem anderen Element von dem Trageelement (2) und dem Schlauchelement (3) befestigt werden kann.

4. Unterstützungsvorrichtung nach einem der Ansprüche 1 oder 2, wobei der Bandabschnitt (5) ein von dem Trageelement (2) und dem Schlauchelement (3) getrenntes Element ist und enthält:
einen ersten Abschnitt (51), der an dem Trageelement (2) oder dem Schlauchelement (3) befestigt werden kann; sowie
einen zweiten Abschnitt (52), der an dem anderen Element von dem Trageelement (2) und dem Schlauchelement (3) befestigt werden kann.

5. Unterstützungsvorrichtung nach einem der Ansprüche 1 bis 4, wobei das Trageelement (2) ein Paar Seitenwände (23) aufweist, zwischen denen ein Teil des Fingers (F) zu positionieren ist, und
eine Höhe (h) der paarigen Seitenwände (23) eine Höhe ist, in der eine Licht-Emissionseinrichtung (E) und ein Licht-Detektor (R) in einer Querschnittsform des Trageelementes (2) positioniert sind.

6. Unterstützungsvorrichtung nach einem der Ansprüche 1 bis 5, wobei eine Haftfläche oder ein Flächen-Haftelement an einer Fläche des Trageelementes (2) oder/und des Schlauchelementes (3) positioniert ist, wobei die Fläche dem Finger (F) gegenüberliegt.

7. Unterstützungsvorrichtung nach einem der Ansprüche 1 bis 6, wobei ein konkaver Abschnitt (22b) an einer Fläche (22a) des Trageelementes (2) ausgebildet ist, und die Fläche (22a) dem Finger (F) gegenüberliegt.

8. Unterstützungsvorrichtung nach einem der Ansprüche 1 bis 7, wobei das Trageelement (2) eine Öffnung (21a) aufweist, durch die ein Teil des Fingers (F) zu sehen ist, wenn das Trageelement (F) an dem Finger (F) angebracht ist.

9. Unterstützungsvorrichtung nach Anspruch 1, wobei das Schlauchelement (3) enthält:
den ersten Abschnitt (31), der aus dem gleichen Kunststoffmaterial wie das Kunststoffmaterial des Trageelementes (2) besteht.

10. Sensor mit Unterstützungsvorrichtung umfassend:
einen Sensor, der zum Erfassen eines physiologischen Parameters des Patienten ausgeführt ist; sowie
eine Unterstützungsvorrichtung nach Anspruch 1.

## Revendications

1. Outil de support (1) qui doit être fixé à un doigt (F) d'un sujet, et qui est utilisé pour soutenir l'acquisition d'un paramètre physiologique du sujet, comprenant :
un élément de support (2) qui doit être placé sur un premier côté du doigt (F) ;
un élément de sac (3) qui doit être placé sur un deuxième côté du doigt (F) opposé au premier côté ;
un passage de fluide (4) qui communique avec l'intérieur de l'élément de sac (3) ; et
une partie de bande (5) qui contraint l'élément de sac (3) au doigt (F)
dans lequel un capteur utilisé pour l'acquisition du paramètre physiologique peut être placé au moins entre le doigt (F) et l'élément de support (2), et entre le doigt (F) et l'élément de sac (3),
**caractérisé en ce que** l'élément de support (2) inclut une partie charnière (21) qui est continue à une première partie (31) de l'élément de sac (3),
dans lequel la partie charnière (21) peut être pliée, et
dans lequel l'élément de sac (3) inclut :
la première partie (31), dans lequel la première partie (31) est moulée d'un seul tenant avec l'élément de support (2) ; et
une deuxième partie (32) qui est soudée à la première partie (31) de sorte que la première partie et la deuxième partie définissent un intérieur de l'élément de sac entre elles.

2. Outil de support selon la revendication 1, dans lequel la deuxième partie (32) est plus mince que la première partie (31).

3. Outil de support selon l'une quelconque des revendications 1 ou 2, dans lequel la partie de bande (5) est moulée d'un seul tenant avec l'un de l'élément de support (2) et de l'élément de sac (3), et peut être fixée à un autre de l'élément de support (2) et de l'élément de sac (3).

4. Outil de support selon l'une quelconque des revendications 1 ou 2, dans lequel la partie de bande (5) est un élément distinct de l'élément de support (2) et de l'élément de sac (3), et inclut :
une première partie (51) qui peut être fixée à l'un de l'élément de support (2) et de l'élément de sac (3) ; et
une deuxième partie (52) qui peut être fixée à un autre parmi l'élément de support (2) et l'élément de sac (3).

5. Outil de support selon l'une quelconque des revendications 1 à 4, dans lequel l'élément de support (2) a une paire de parois latérales (23) entre lesquelles une partie du doigt (F) est destinée à être placée, et
une hauteur (h) de la paire de parois latérales (23) est une hauteur à laquelle un émetteur de lumière (E) et un détecteur de lumière (R) sont positionnés dans une forme de section transversale de l'élément de support (2).

6. Outil de support selon l'une quelconque des revendications 1 à 5, dans lequel une surface adhésive ou une fixation de surface est placée sur une surface d'au moins l'un de l'élément de support (2) et de l'élément de sac (3), la surface étant opposée au doigt (F).

7. Outil de support selon l'une quelconque des revendications 1 à 6, dans lequel une partie concave (22b) est formée sur une surface (22a) de l'élément de support (2), la surface (22a) étant opposée au doigt (F).

8. Outil de support selon l'une quelconque des revendications 1 à 7, dans lequel l'élément de support (2) a une ouverture (21a) à travers laquelle une partie du doigt (F) est vue lorsque l'élément de support (F) est fixé au doigt (F).

9. Outil de support selon la revendication 1, dans lequel l'élément de sac (3) inclut :
la première partie (31) étant formée par le même matériau de résine que le matériau de résine de l'élément de support (2).

10. Capteur avec un outil de support comprenant :
un capteur configuré pour acquérir des paramètres physiologiques du sujet ; et
un outil de support selon la revendication 1.
